# EUROPÄISCHE PATENTSCHRIFT

(11) **EP 0 769 497 B1**
(45) Veröffentlichungstag und Bekanntmachung des Hinweises auf die Patenterteilung: **18.12.2002**
(21) Anmeldenummer: 96116152.8
(22) Anmeldetag: 09.10.1996
(51) Int. Cl.: C07D 311/72

(54) **Hydroxymethylierung von Tocopherolen**
Hydroxymethylation of Tocopherols
Hydroxyméthylation de tocophéroles

(30) Priorität: 18.10.1995 CH 295195
(43) Veröffentlichungstag der Anmeldung: 23.04.1997
(73) Patentinhaber: F. HOFFMANN-LA ROCHE AG, 4070 Basel (CH)
(72) Erfinder: Brüggemann, Konrad, 4323 Wallbach (CH); Herguijuela, Juan Ramon, 79424 Auggen (DE); Netscher, Thomas, 79189 Bad Krozingen (DE); Riegl, Johann, 79110 Freiburg (DE)
(74) Vertreter: Kellenberger, Marcus, Dr.

(56) Entgegenhaltungen:
- EP-A- 0 178 400
- EP-A- 0 191 132
- US-A- 3 819 657
- DATABASE WPI Derwent Publications Ltd., London, GB; AN 76-56625x XP002023411 & JP 60 004 183 A (EISAI)

## Beschreibung

Die vorliegende Erfindung betrifft die Hydroxymethylierung von Gemischen, welche sogenannte "Nicht-α-Tocopherole" enthalten, und die anschliessende Ueberführung der Hydroxymethylierungsprodukte in α-Tocopherol, wobei die beiden Reaktionsstufen gemeinsam als Eintopfverfahren erfolgen.

Bekanntlich unterscheiden sich die natürlich vorkommenden Nicht-α-Tocopherole β-, γ- und δ-Tocopherol von dem die höchste Vitamin E-Aktivität aufweisenden und somit biologisch wertvollsten Tocopherol, α-Tocopherol, durch das Fehlen einer oder zweier Methylgruppen in 5- und/oder 7-Stellung des Chromanteils des Moleküls. Daher besteht ein Bedürfnis, derartige Nicht-α-Tocopherole in das α-Tocopherol chemisch überzuführen, wobei das Hauptproblem an der effizienten, ergänzenden Mono- bzw. Dimethylierung des Benzolrings der substituierten Chromanylgruppe liegt.

Da sich synthetische Verfahren zur Herstellung von naturidentischem α-Tocopherol bislang als unwirtschaftlich erwiesen haben und natürliche, insbesondere pflanzliche Quellen von Tocopherolen in der Regel nur einen relativ geringen Anteil an α-Tocopherol, hingegen überwiegend Nicht-α-Tocopherole, enthalten, so dass auch die Isolierung des α-Tocopherols aus solchen natürlichen Materialien (Rohmaterialien) ebenfalls eher unrentabel ist, besteht die Aufgabe der vorliegenden Erfindung darin, ein Verfahren zur Umwandlung von eventuell in einschlägigen Rohmaterialien vorhandenen oder aus diesen gewonnenen Nicht-α-Tocopherolen in α-Tocopherol zu entwickeln, welches in vielerlei Hinsicht wirtschaftlicher ist als die bisherigen einschlägigen Verfahren.

Im Sinne dieser Erfindungsaufgabe sind bereits einige Verfahren zur Umwandlung von Nicht-α-Tocopherolen in α-Tocopherol aus dem Stande der Technik bekannt geworden. Beispielsweise offenbart die japanische Patentpublikation (Kokoku) Nr. 4183/1985 (Eisai Co. Ltd.) ein Verfahren zur Herstellung von α-Tocopherol aus Nicht-α-Tocopherolen durch Umsetzung mindestens eines Nicht-α-Tocopherols mit Formaldehyd in Gegenwart von Borsäure oder eines Derivats davon unter katalytisch reduzierenden Bedingungen. Bei diesem Verfahren, das sowohl eine Hydroxymethylierung als auch eine anschliessende Reduktion beinhaltet, handelt es sich um ein Eintopfverfahren, so dass die Isolierung der sehr instabilen hydroxymethylierten Zwischenprodukte sowie Nebenreaktionen umgangen werden. Hingegen wird das Verfahren offenbar bei relativ hohen Temperaturen von etwa 200°C durchgeführt (siehe Beispiele 1-12), was erfahrungsgemäss zwangsläufig zu unerwünschter Zersetzung des Formaldehyds zu Kohlenmonoxid und Wasserstoff und demzufolge zu einem Druckanstieg im Reaktionsgefäss führt. Ferner wird bemerkt, dass eine ganze Reihe von vermeintlich geeigneten Lösungsmitteln, u.a. auch das Reagens "Alkylborat" (Trialkylborat, z.B. Trimethylborat), erwähnt ist; hingegen wird die Möglichkeit der Verwendung einer Kombination von Lösungsmitteln nicht angedeutet. Wegen der Verwendung von relativ hohen Temperaturen bei dem bekannten Verfahren bedingt diese stärkere und infolgedessen teure Verfahrensanlagen (Autoklaven) sowie hohe Energiekosten beim Aufheizen und Abkühlen. Insbesondere bringt der durch die Zersetzung des Formaldehyds verursachte Druckanstieg Probleme, nicht zuletzt Gefahren, bei der Reaktionsführung: die dabei erzeugten Gase machen teure Sicherheitsmassnahmen notwendig, was die Wirtschaftlichkeit reduziert und zusätzliche Investitionen erforderlich macht. Ferner werden bei Verwendung von Trimethylborat als alleinigem Lösungsmittel die besten Ergebnisse erzielt; diese Verwendung ist jedoch aufwendig in der Herstellung bzw. Recyclisierung und stellt somit eine teure Massnahme dar. Daher weist das in der japanischen Patentpublikation Nr. 4183/1985 offenbarte Verfahren schwerwiegende Nachteile hinsichtlich Investitionsund Betriebskosten einer Anlage auf.

Es wurde nun überraschenderweise gefunden, dass dieses bekannte Verfahren der Eisai Co. Ltd. durch eine ganz besondere Wahl der Reaktionsbedingungen entscheidend verbessert werden kann. Gegenstand der vorliegenden Erfindung ist ein Verfahren zur Umwandlung von Nicht-α-Tocopherolen in α-Tocopherol durch Umsetzung (Hydroxymethylierung) mindestens eines Nicht-α-Tocopherols mit Formaldehyd oder einem Derivat davon in Gegenwart von Borsäure oder einem Derivat davon unter katalytisch reduzierenden Bedingungen, das dadurch gekennzeichnet ist, dass man als Lösungsmittel mit der zusätzlichen Funktion eines Katalysators ein azeotropes Gemisch von Trimethylborat und Methanol sowie als weiteres Lösungsmittel ein nichtpolares organisches Lösungsmittel verwendet, und dass man die Umsetzung bei Temperaturen zwischen 130 und 180°C durchführt.

Wie bereits oben angedeutet, kann im Prinzip als Edukt im erfindungsgemässen Verfahren ein Rohmaterial, das zumindest ein Nicht-α-Tocopherol, z.B. β-, γ- oder δ-Tocopherol, enthält, oder ein aus einem solchen Rohmaterial gewonnenes oder anderweitig erhaltenes Tocopherolgemisch, das ebenfalls zumindest ein Nicht-α-Tocopherol enthält, eingesetzt werden. Da bekanntlich pflanzliche Oele und Fette, wie beispielsweise Sojaöl, Rapsöl, Baumwollsaatöl, Erdnussöl, Weizenkeimöl, Maisöl, Gerstenöl, Roggenöl, Distelöl und dergleichen, wertvolle natürliche Quellen von Tocopherolen (u.a. α- und Nicht-α-Tocopherole) sind, können solche Oele oder vorzugsweise deren Destillate, die einen höheren Gehalt an Tocopherolen aufweisen und weniger unerwünschte andere Bestandteile, z.B. Sterole, freie und veresterte Fettsäuren, Wachse und Glyzeride, enthalten, als Edukt im erfindungsgemässen Verfahren Verwendung finden. Insbesondere Distelöl und Sojaöl erweisen sich als wertvolle Quellen von Tocopherolen, u.a. α-Tocopherol und den in dieses erfindungsgemäss überzuführenden Nicht-α-Tocopherolen. Es spielt natürlich keine Rolle, dass sich u.a. α-Tocopherol selber im Edukt befindet, da das α-Tocopherol die Ueberführung der Nicht-α-Tocopherole in α-Tocopherol nicht verhindert und selber unreagiert im Verfahrensprodukt bleibt.

Unter dem Ausdruck "Derivat" in bezug auf den im erfindungsgemässen Verfahren als Reagens verwendeten Formaldehyd versteht sich insbesondere ein Oligomer oder Oligomerengemisch von Formaldehyd, wie beispielsweise Paraformaldehyd, oder ein Formaldehyd abspaltendes Kondensationsprodukt von Formaldehyd, wie beispielsweise Formaldehyddimethylacetal. Gewünschtenfalls kann man den Formaldehyd als gasförmigen Formaldehyd einsetzen. Vorzugsweise verwendet man allerdings den festen Paraformaldehyd. Die anzuwendende Menge an Formaldehyd (als solchem oder als Anteil des Derivats) liegt geeigneterweise zwischen etwa 2 und 10 Moläquivalenten, vorzugsweise zwischen etwa 4 und etwa 8 Moläquivalenten, pro Moläquivalent (totale) Nicht-α-Tocopherol(e).

Ein weiteres Merkmal des erfindungsgemässen Verfahrens besteht darin, dass man die Hydroxymethylierung in Gegenwart von Borsäure oder einem Derivat davon durchführt. Im letzteren Fall eignet sich als Derivat insbesondere das ohnehin im Reaktionsgemisch vorhandene Trimethylborat, wobei man dann vorzugsweise auch noch Wasser zugibt, um mit dem Trimethylborat die Borsäure (in situ) zu erzeugen. Falls Borsäure selber eingesetzt wird, so beträgt deren Menge geeigneterweise etwa 0,1 bis etwa 4 Moläquivalente, vorzugsweise etwa 0,5 bis etwa 1 Moläquivalent, pro Moläquivalent α-Tocopherol und Nicht-α-Tocopherole (Gesamt-Tocopherole). Andernfalls, d.h. bei Verwendung des bereits vorhandenen Trimethylborats mitsamt zugesetztem Wasser, verwendet man zweckmässigerweise etwa 0,3 bis etwa 12 Moläquivalente, vorzugsweise etwa 1,5 bis etwa 3 Moläquivalente, Wasser pro Moläquivalent Gesamt-Tocopherole.

Ferner wird die Hydroxymethylierung unter katalytisch reduzierenden Bedingungen durchgeführt. In der Praxis heisst dies, dass die Hydroxymethylierung mittels Formaldehyd oder eines Derivates davon sowie die unmittelbar anschliessende Hydrierung des hydroxymethylierten Tocopherols bzw. Tocopherolgemisches gemeinsam im selben Reaktionsgefäss erfolgen, ohne dass die Isolierung des hydroxymethylierten Produktes benötigt wird. Zum Zwecke der Hydrierung verwendet man als Katalysator einen gängigen Edelmetallkatalysator, z.B. einen auf Palladium oder Platin basierenden Hydrierungskatalysator, wie beispielsweise Palladium auf Aktivkohle oder Platin auf Aktivkohle. Vorzugsweise wird Palladium auf Aktivkohle mit einem 5- bis 10-prozentigen Palladiumanteil verwendet. Die Hydrierung benötigt zweckmässigerweise einen Wasserstoffdruck von etwa 10 bar bis etwa 75 bar, vorzugsweise einen von etwa 25 bar bis etwa 35 bar.

Das im erfindungsgemässen Verfahren als Lösungsmittel mit der zusätzlichen Funktion eines Katalysators - und für die Erfindung kennzeichnend - verwendete "azeotrope" Gemisch von Trimethylborat und Methanol weist zwangsläufig ein gewisses Gewichtsverhältnis auf. Dieses Verhältnis (Trimethylborat:Methanol) liegt im allgemeinen in der Nähe des Azeotrops, d.h. bei 50:50 bis 75:25, vorzugsweise 70:30. Bezogen auf die eingesetzte Menge Gesamt-Tocopherole beträgt das Moläquivalentenverhältnis Trimethylborat:Gesamt-Tocopherole zweckmässigerweise 0,1:1 bis 10:1, vorzugsweise 1:1 bis 4:1.

Ebenfalls für die Erfindung kennzeichnend wird ein weiteres Lösungsmittel verwendet, und zwar ein nichtpolares organisches Lösungsmittel. Dieses ist vorzugsweise ein niederes (insbesondere C₅₋₈-)Alkan, ein Gemisch von Alkanen (Petrolether) oder ein aromatischer Kohlenwasserstoff, z.B. Toluol. Besonders bevorzugt verwendet man ein niederes Alkan, insbesondere n-Hexan. Geeigneterweise verwendet man pro Moläquivalent Gesamt-Tocopherole bis zu 10 Liter nichtpolares organisches Lösungsmittel, vorzugsweise etwa 1 bis etwa 3 Liter.

Im Prinzip wird das erfindungsgemässe Verfahren so durchgeführt, dass man das obenerwähnte Rohmaterial bzw. Tocopherolgemisch, den Formaldehyd bzw. das Derivat davon, die Lösungsmittel (azeotropes Gemisch von Trimethylborat und Methanol sowie nichtpolares organisches Lösungsmittel), die Borsäure oder das Wasser, sowie den Hydrierungskatalysator im Reaktionsgefäss vorlegt und den Wasserstoff einleitet, bis der Hydrierungsdruck erreicht wird. Dann wird das Reaktionsgemisch vorzugsweise unter Rühren aufgeheizt. Die Umsetzung wird bei Temperaturen zwischen 130 und 180°C, vorzugsweise bei Temperaturen zwischen 140 und 170°C, durchgeführt, und dauert in der Regel zwischen 4 und 8 Stunden. Der Reaktionsablauf kann unter Verwendung von chromatographischen Methoden, z.B. Gaschromatographie (GC), verfolgt werden und nach Feststellung beendeter Reaktion das erhaltene α-Tocopherol, welches in der Regel frei von anderen Nicht-α-Tocopherol-Homologen (β-, γ-, δ-Tocopherol) ist, vom verbleibenden Gemisch gewünschtenfalls nach an sich bekannten Methoden isoliert und dann nötigenfalls gereinigt werden. Unter anderem kann dabei der Katalysator, beispielsweise nach Spülen mit einem geeigneten organischen Lösungsmittel, z.B. Methanol, erneut eingesetzt werden.

Die vorliegende Erfindung umfasst als weiteren Aspekt die Aufarbeitung des Gemisches nach Beendung der Reaktion, bei der es sich nicht nur um die Isolierung des α-Tocopherols handelt, sondern auch um eine nahezu vollständige Abtrennung aller bei der Umsetzung verwendeten Lösungsmittel-Komponenten, des Katalysators und, im Falle der Verwendung von Borsäure, von dieser bzw. dem Trimethylborat, so dass die zurückerhaltenen Substanzen wiederverwendet (recyclisiert) werden können. Die Aufarbeitung ist ökonomisch und daher vorteilhaft.

Für die Aufarbeitung kennzeichnend ist, dass die Rückführung der Borsäure bzw. des Trimethylborats einzig in Form eines Trimethylborat-Methanol-Gemisches durch rektifikative Destillation erfolgt. Wie oben erwähnt, kann die für die im erfindungsgemässen Verfahren stattfindende Reaktion benötigte Borsäure als Borsäure selbst zugesetzt werden oder aus dem im Reaktionsgemisch (als Teil des azeotropen Gemisches) vorhandenen Trimethylborat durch Wasserzusatz in situ erzeugt werden. Die Aufarbeitung besteht darin, dass man das nach beendeter Reaktion verbleibende Gemisch mit Methanol behandelt, wobei sich die Borsäure im Methanol löst, die so erhaltene, gewünschtenfalls durch Spülungen des nichtpolaren organischen Lösungsmittels ergänzte, verbleibende Lösung vom festen Katalysator abfiltriert, welcher so isoliert und wiederverwendet werden kann, das (gesamte) Filtrat mit Wasser behandelt, nach Phasentrennung das nichtpolare organische Lösungsmittel aus der organischen Phase destillativ zur Wiederverwendung entfernt, um das Reaktionsprodukt α-Tocopherol freizusetzen, und die hauptsächlich aus Methanol, Wasser und Borsäure bestehende wässrige Phase einer sogenannten Reaktiv-Destillation unterwirft, um das bei der Destillation infolge der Veresterung von Borsäure mit Methanol erzeugte Trimethylborat als "azeotropes" Gemisch von Trimethylborat und Methanol abzutrennen und zusätzlich Methanol und Wasser einer Wiederverwendung zuzuführen. Da bekanntlich Borsäurealkylester sehr leicht hydrolysiert werden, ist es überraschend, dass aus Mischungen von Borsäure, Wasser und Methanol mit einem Wassergehalt von über 30% "azeotrope" Gemische von Trimethylborat und Methanol gewonnen werden können. Unter Verwendung dieser Aufarbeitung können das hauptsächlich aus α-Tocopherol bestehende Reaktionsprodukt isoliert und das Methanol, das Wasser, das nichtpolare organische Lösungsmittel, der Hydrierkatalysator sowie die Borsäure als "azeotropes" Gemisch von Trimethylborat und Methanol isoliert und wiederverwendet (recyclisiert) werden.

Die im erfindungsgemässen Verfahren verwendete Lösungsmittelkombination des (kommerziell erhältlichen) azeotropen Gemisches von Trimethylborat und Methanol mit einem nichtpolaren Lösungsmittel, vorzugsweise mit n-Hexan, ermöglicht im Vergleich zum Stande der Technik deutlich günstigere Reaktionsbedingungen bei ebenso hohen Ausbeuten an α-Tocopherol. Dieses Ergebnis ist deswegen überraschend, da sich die Resultate unter Verwendung jedes Lösungsmittels einzeln nicht erreichen lassen, indem beispielsweise schlechtere Umsätze, vermehrt Nebenprodukt-Bildung oder eine höhere erforderliche Reaktionstemperatur in Kauf genommen werden müssen. Daraus ergeben sich u.a. folgende ökonomisch nutzbare Vorteile des erfindungsgemässen Verfahrens:
1) Durch die niedrigere Reaktionstemperatur:
   - geringere Zersetzung von Formaldehyd, und dadurch niedrigerer Formaldehyd-Verbrauch, vereinfachte Reaktionsführung [beispielsweise als Folge keiner (übermässigen) Druckbildung] und Vermeidung zusätzlicher teurer Sicherheitsmassnahmen;
   - kostengünstigere Verfahrensanlagen und niedrigere Energiekosten, d.h. geringere Investitions- und Betriebskosten;
   - raschere Aufheiz- und Abkühlphasen bei der Verfahrensdurchführung; sowie
   - geringere thermische Belastung von Edukt und Produkt (Tocopherolen).
2) Durch die Verwendung des azeotropen Gemisches:
   - geringere Betriebskosten durch die Verwendbarkeit des gegenüber Trimethylborat wesentlich billigeren azeotropen Gemisches von Trimethylborat und Methanol;
   - (weitgehende) Vermeidung der Handhabung fester Borsäure, was ein kostengünstiges Verfahren ermöglicht sowie
   - vollkontinuierliche Recyclisierung der Bor-Reagenzien (bei der Reaktiv-Destillation) und Lösungsmittel, was wiederum ein kostengünstiges (ökonomisch durchführbares) Verfahren ermöglicht.

Die vorliegende Erfindung wird durch die nachfolgenden Beispiele veranschaulicht:

### Beispiel 1

### Hydroxymethylierung-Hydrierung von δ-Tocopherol (Eintopfverfahren)

In einem 500 ml-Stahlrührautoklav mit mechanischem Begasungsrührer werden 25,75 g d-δ-Tocopherol [der Firma Sigma; etwa 90%ig, Gehalt an δ-Tocopherol 84,6%, 54,1 mmol; γ-Tocopherol 5,3%, 3,3 mmol; α-Tocopherol 1,3%, 0,8 mmol; Gesamt-Tocopherole 91,2%, 58,2 mmol; bestimmt mittels GC der Acetate, interner Standard Squalan; stereochemische Reinheit: >99,5% R,R,R-Isomere (HPLC des Methyläther-Derivates, bestimmt an Chiracel OD, einer käuflichen Chromatographie(HPLC)-Säule von Daicel)], 80 ml n-Hexan, 35,4 g Trimethylborat-Methanol-Azeotrop (Trimethylborat-Gehalt 70 Gewichts-%, entsprechend 24,8 g Trimethylborat), 14,0 g Paraformaldehyd (entsprechend 466 mmol, 8 Moläquivalente, bezogen auf Gesamt-Tocopherole), 1,57 ml Wasser (ionenarm, 87,2 mmol, zur Erzeugung von 29,1 mmol Borsäure, 0,5 Moläquivalente bezogen auf Gesamt-Tocopherole) und 2,5 g Palladium auf Aktivkohle (10%ig, Degussa E10N/D) vereinigt. Nach Verschliessen wird der Autoklav ohne Rühren durch dreimaliges Aufdrücken von etwa 10 bar Wasserstoff und nachfolgendes Entlasten gespült. Man drückt dann unter Rühren bei 22°C 15 bar Wasserstoff auf. Unter Rühren (300 U/ Min.) wird während etwa 15 Minuten auf 161°C aufgeheizt. Hierbei erfolgt ein Druckanstieg auf 26,6 bar. Es wird nochmals Wasserstoff bis zu einem Druck von 30 bar aufgedrückt und 7 Stunden bei 159 bis 160°C und offenem Wasserstoffventil gerührt, wobei sich ein Druck zwischen 29,7 und 31,3 bar einstellt. Man lässt eine Stunde auf 20°C abkühlen. Nach Öffnen des Autoklaven wird das Reaktionsgemisch über eine Fritte vom Katalysator getrennt, und der feste Rückstand (Katalysator und Borsäure) dreimal mit je 100 ml n-Hexan gewaschen (sofern eine Recyclisierung der Borsäure durch Reaktiv-Destillation entsprechend Beispiel 4 gewünscht ist, wäscht man den Rückstand zusätzlich mit Methanol). Die organischen Filtrate werden vereinigt und unter vermindertem Druck von 400 mbar und bei einer Badtemperatur von 60°C von den leichtflüchtigen Anteilen befreit. Der erhaltene rotbraune, klare, manchmal leicht trübe Destillationsrückstand wird in 250 ml n-Hexan aufgenommen, und das Gemisch in einem 500 ml-Rundkolben mit 150 ml Wasser als zweiphasiges Gemisch 30 Minuten bei 55-60°C Badtemperatur gerührt. Hierbei erfolgt die Hydrolyse der Borester. Die Hexanphase wird in einem 500 ml-Scheidetrichter einmal mit 150 ml Wasser gewaschen, um die Borsäure zu entfernen, die Hexanphase abgetrennt und über 3 g wasserfreiem Magnesiumsulfat 10 Minuten unter Rühren getrocknet. Nach Filtration und Abdestillieren des Lösungsmittels bei 400 mbar und einer Badtemperatur von 60°C und anschliessender 30-minütiger Trocknung bei 15 mbar und einer Badtemperatur von 60-70°C erhält man 28,62 g rohes α-Tocopherol als gelbes bis hellbraunes Öl; Gehalt an α-Tocopherol (GC der Acetate, interner Standard Squalan) 81,5%, chemische Ausbeute (α-Tocopherol) 93,1%, keine anderen Homologen (β-, γ-, δ-Tocopherol), 7-Hydroxymethyl-β-Tocopherol 1,6%, Bor-Gehalt <10 ppm. Die abdestillierten Lösungsmittel aus der organischen Phase bestehen vorwiegend aus n-Hexan und lassen sich umgehend recyclisieren. Die vereinigten wässrigen Phasen enthalten Methanol, Wasser und Borsäure und können einer destillativen Aufarbeitung zugeführt werden (vergleiche Beispiel 4). Das rohe α-Tocopherol wird mit 25,0 g Essigsäureanhydrid (entsprechend 244,9 mmol), 25,0 g Pyridin (entsprechend 316,1 mmol) und 1,0 g 4-Dimethylaminopyridin (entsprechend 8,2 mmol; katalytische Menge) versetzt und eine Stunde bei Raumtemperatur gerührt. Man giesst das Reaktionsgemisch auf 100 g Eis/ Wasser (etwa 1:1), rührt 30 Minuten, versetzt mit 250 ml n-Hexan und wäscht nacheinander zweimal mit je 150 ml Wasser, zweimal mit je 100 ml 2N Schwefelsäure, mit 150 ml Wasser, mit 150 ml gesättigter Natriumbicarbonat-Lösung und dreimal mit je 150 ml Wasser. Danach wird über 3 g wasserfreiem Magnesiumsulfat 10 Minuten unter Rühren getrocknet. Nach Filtration, Abdestillieren des Lösungsmittels bei 400 mbar und einer Badtemperatur von 60° C und anschliessender 30-minütiger Trocknung bei 15 mbar und einer Badtemperatur von 70° C erhält man 29,26 g α-Tocopherylacetat als gelbes Öl, Gehalt an α-Tocopherylacetat (GC, interner Standard Squalan) 87,5%, chemische Ausbeute (α-Tocopherylacetat) 93,1%. Dieses Rohacetat wird im Kugelrohrofen destilliert (250°C/ 10⁻¹ mbar). Man erhält 26,25 g α-Tocopherylacetat als schwach gelbliches Öl, das beim Stehen bei Raumtemperatur langsam kristallisiert. Gehalt an α-Tocopherylacetat (GC, interner Standard Squalan) 96,2%, chemische Ausbeute 91,8%; stereochemische Reinheit: 99,6% R,R,R-α-Isomer (HPLC des Methylether-Derivates an Chiracel OD, n-Hexan); das Produkt ist gemäss ¹H-NMR-, IR-, MS- und Mikroanalyse-Daten mit α-Tocopherylacetat identisch.

### Beispiel 2

### Hydroxymethylierung-Hydrierung eines Tocopherolgemisches (Eintopfverfahren)

In einem 500 ml-Stahlrührautoklav mit mechanischem Begasungsrührer werden 25,75 g eines Tocopherol-Gemisches aus natürlichen Quellen (bestehend aus α-Tocopherol 3,9%, 2,3 mmol; β-Tocopherol 1,0%, 0,6 mmol; γ-Tocopherol 60,6%, 37,5 mmol; δ-Tocopherol 26,4%, 16,9 mmol; Gesamt-Tocopherole 91.9%, 57.3 mmol; bestimmt mittels GC der Acetate, interner Standard Squalan), 80 ml n-Hexan, 34,9 g Trimethylborat-Methanol-Azeotrop (Trimethylborat-Gehalt 70 Gewichts-%, entsprechend 24,4 g Trimethylborat), 10,3 g Paraformaldehyd (entsprechend 344 mmol, 6 Moläquivalente, bezogen auf Gesamt-Tocopherole), 1,57 ml Wasser (ionenarm, 87,2 mmol, zur Erzeugung von 29,1 mmol Borsäure, 0,5 Moläquivalente bezogen auf Gesamt-Tocopherole) und 2,5 g Palladium auf Aktivkohle (10%ig, Degussa E10N/D) vereinigt. Nach Verschliessen wird der Autoklav ohne Rühren durch dreimaliges Aufdrücken von etwa 10 bar Wasserstoff und nachfolgendes Entlasten gespült. Man drückt dann unter Rühren bei 22°C 15 bar Wasserstoff auf. Unter Rühren (300 U/ Min.) wird während etwa 15 Minuten auf 163°C aufgeheizt. Hierbei erfolgt ein Druckanstieg auf 27,5 bar. Es wird nochmals Wasserstoff bis zu einem Druck von 30 bar aufgedrückt und 7 Stunden bei 159-160°C und offenem Wasserstoffventil gerührt, wobei der Druck zwischen 29,6 und 32,2 bar liegt. Man lässt eine Stunde auf 50°C abkühlen, ersetzt den Wasserstoff durch Stickstoff, öffnet den Autoklaven und gibt zum Reaktionsgemisch unter Rühren 100 ml Methanol. Man verschliesst den Autoklaven nochmals, rührt 30 Minuten bei 50°C und lässt dann 45 Minuten auf 23°C abkühlen. Nach Öffnen des Autoklaven wird das Reaktionsgemisch über eine Fritte vom Katalysator getrennt, und der feste Rückstand (Katalysator) dreimal mit je 100 ml n-Hexan gewaschen. Die vereinigten Filtrate bilden ein Zweiphasengemisch. Unter vermindertem Druck von 400 mbar und bei einer Badtemperatur von 60°C werden alle leichtflüchtigen Anteile abdestilliert. Der gelbe bis hellbraune Destillationsrückstand enthält geringe Mengen kristalliner Borsäure. Der Rückstand wird in 250 ml n-Hexan aufgenommen, und das Gemisch in einem 500 ml-Scheidetrichter zweimal mit je 150 ml Wasser gewaschen, wobei die Borsäure entfernt wird. Die Hexanphase wird abgetrennt und über 3 g wasserfreiem Magnesiumsulfat 10 Minuten unter Rühren getrocknet. Nach Filtration und Abdestillieren des Lösungsmittels bei 400 mbar und einer Badtemperatur von 60°C und anschliessender, 30-minütiger Trocknung bei 15 mbar und einer Badtemperatur von 60-70°C erhält man 26,91 g rohes α-Tocopherol als gelbes bis hellbraunes Öl; Gehalt an α-Tocopherol (GC der Acetate, interner Standard Squalan) 88,45%, chemische Ausbeute (α-Tocopherol) 96,5%, keine anderen Homologen (β-, γ-, δ-Tocopherol), 7-Hydroxymethyl-β-Tocopherol 0,6%, Bor-Gehalt 11 ppm. Die Lösungsmittel und Reagenzien können nach Bedarf wie in Beispiel 1 angegeben einer Recyclisierung zugeführt werden. Das rohe α-Tocopherol wird analog Beispiel 1 acetyliert, um 29,89 g rohes α-Tocopherylacetat als gelbes Öl zu liefern; Gehalt an α-Tocopherylacetat (GC, interner Standard Squalan) 87,1%, chemische Ausbeute (α-Tocopherylacetat) 96,1 %. Dieses Rohacetat wird im Kugelrohrofen destilliert (250°C/ 10⁻¹ mbar); man erhält 27,25 g α-Tocopherylacetat als schwach gelbliches Öl, das beim Stehen bei Raumtemperatur langsam kristallisiert. Gehalt an α-Tocopherylacetat (GC, interner Standard Squalan) 94,7%, chemische Ausbeute 95,3%; stereochemische Reinheit 99,6 % R,R,R-α-Isomer (HPLC des Methylether-Derivates an Chiracel OD, n-Hexan); das Produkt ist gemäss ¹H-NMR-, IR-, MS- und Mikroanalyse-Daten mit α-Tocopherylacetat identisch.

### Beispiel 3

### Hydroxymethylierung-Hydrierung eines Tocopherol-Homologen-Gemisches mit anschließender Methanolyse (Eintopfverfahren); Zurückgewinnung der Verfahrenskomponenten

In einem 500 ml-Stahlrührautoklav mit mechanischem Begasungsrührer werden 25,75 g Tocopherol-Homologen-Gemisch aus natürlichen Quellen [bestehend aus α-Tocopherol 3,9%, 2,3 mmol; β-Tocopherol 1,0%, 0,6 mmol; γ-Tocopherol 60,6%, 37,5 mmol; δ-Tocopherol 26,4%, 16,9 mmol; Gesamt-Tocopherole 91,9%, 57,3 mmol; bestimmt mittels GC der Acetate, interner Standard Squalan], 100 ml n-Hexan, 30,6 g Trimethylborat-Azeotrop in Methanol (Trimethylborat-Gehalt 70 Gewichts-%, entsprechend 21,4 g Trimethylborat), 10,3 g Paraformaldehyd (entsprechend 344 mmol, 6 Mol-Equiv., bezogen auf Gesamt-Tocopherole), 3,1 ml Wasser (ionenarm, 172 mmol, zur Erzeugung von 57,3 mmol Borsäure, 1,0 Mol-Equivalente bezogen auf Gesamt-Tocopherole) und 2,5 g Palladium auf Aktivkohle (10%ig, Degussa E10N/D)) vereinigt. Nach Verschliessen des Autoklaven drückt man ca. 10 bar Wasserstoff auf und entspannt anschließend. Man wiederholt diesen Vorgang dreimal, drückt dann nochmals 10 bar Wasserstoff auf und heizt unter Rühren (300 U/ Min.) während ca. 30 Minuten auf 160°C auf. Hierbei erfolgt ein Druckanstieg auf 23,7 bar. Dann drückt man nochmals Wasserstoff bis zu einem Druck von 30 bar auf und rührt 5 Stunden bei 159-161°C und offenem Wasserstoffventil, wobei der Druck zwischen 30,1 und 31,2 bar liegt. Man lässt dann während 30 Minuten auf 43°C abkühlen, entspannt und drückt ohne Öffnen des Autoklaven 80 g Methanol hinein. Man heizt bei geschlossenem Autoklaven unter Rühren während 15 Minuten auf 162°C (der Innendruck beträgt 21,6 bar) und lässt dann während 15 Minuten auf 20°C abkühlen. Nach Öffnen des Autoklaven wird das Reaktionsgemisch über eine Fritte mit 1 g Speedex (Filterhilfsmittel) vom Katalysator getrennt, und der feste Rückstand (Palladium auf Aktivkohle) mit drei gleichen Portionen, insgesamt 100 ml, n-Hexan gewaschen. Die vereinigten Filtrate bilden ein Zwei-Phasengemisch von 300 ml Gesamtvolumen, welches sich zusammensetzt aus 165 ml einer oberen, gelbgrünen Hexan-Phase und 135 ml einer unteren, farblosen Methanol-Phase. Dieses Gemisch wird mit 70 ml Wasser versetzt, 10 Minuten kräftig gerührt und im Scheidetrichter getrennt. Die untere farblose Phase (205 ml) enthält Wasser, Methanol und Borsäure und wird zur Rückgewinnung der Komponenten der "Reaktiv-Destillation" (siehe Beispiel 4) zugeführt. Aus der oberen gelben organischen Phase (165 ml) destilliert man bei 400 mbar und einer Badtemperatur von 60°C das Lösungsmittel ab (Rückführung des Hexans gemäß nachfolgender Abbildungen 1 und 2) und trocknet bis zur Gewichtskonstanz: 27,78 g rohes α-Tocopherol als gelbes bis hellbraunes Öl, Gehalt (GC der Acetate, interner Standard Squalan) α-Tocopherol 85,9%, chemische Ausbeute (α-Tocopherol) 96,7%, keine anderen Homologen (β-, γ-, δ-Tocopherol), 7-Hydroxymethyl-β-Tocopherol 1,1% [in einem weiteren Ansatz werden 27,01 g Rohprodukt mit einem Gehalt von 87,3% α-Tocopherol erhalten, chemische Ausbeute 95,6% α-Tocopherol, keine anderen Homologen (β-, γ-, δ-Tocopherol), 7-Hydroxymethyl-β-Tocopherol 0,6%]. Das rohe α-Tocopherol wird wie in Beispiel 1 acetyliert: 30,18 g rohes α-Tocopherylacetat als gelbes Öl, Gehalt (GC, interner Standard Squalan) α-Tocopherylacetat 85,6%, chemische Ausbeute (α-Tocopherylacetat) 95,4 %. Dieses Rohacetat wird in einem Kugelrohrofen destilliert (250°C/ 10⁻¹ mbar): man erhält 26,73 g α-Tocopherylacetat als schwach gelbliches Öl, das beim Stehen bei Raumtemperatur langsam kristallisiert. Gehalt (GC, interner Standard Squalan) α-Tocopherylacetat 95,6%, chemische Ausbeute 94,3%; stereochemische Reinheit: 99,5 % R,R,R-α-Isomer (HPLC des Methylether-Derivates an Chiracel OD, Hexan); das Produkt ist gemäß ¹H-NMR, IR, MS und Mikroanalyse mit α-Tocopherylacetat identisch. Das hier beschriebene Herstellungsverfahren, die Aufarbeitung, die Rückgewinnung der Verfahrenskomponenten und deren Recyclisierung für einen neuen Ansatz werden in der nachfolgenden Abbildung 1 schematisch dargestellt, in der zum grossen Teil die diesbezüglichen chemischen Symbole verwendet sind und Me für Methyl steht:

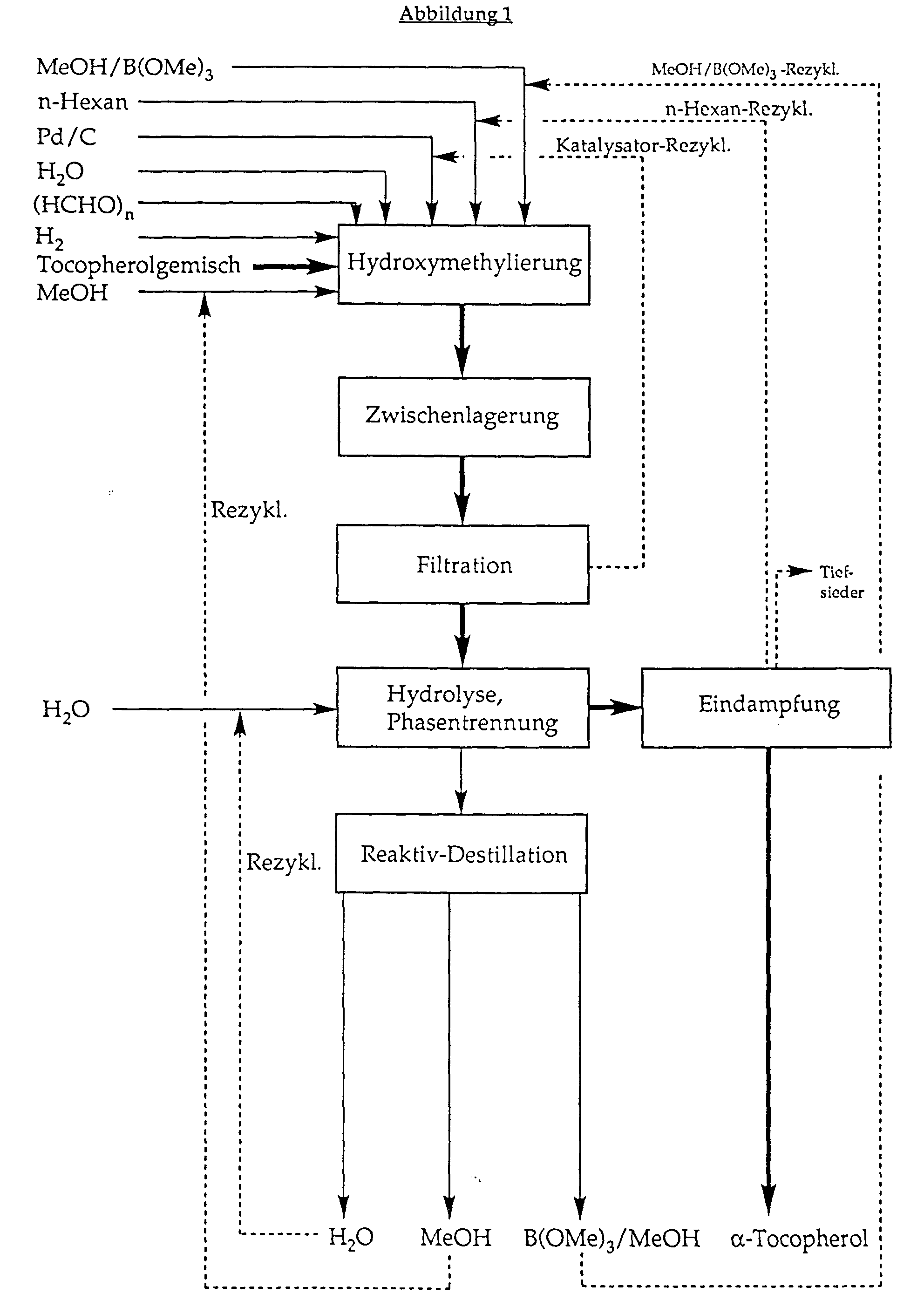

Die nachfolgende Abbildung 2, in der ebenfalls die diesbezüglichen chemischen Symbole verwendet sind und Me für Methyl steht, stellt eine Verfahrensanlage für die Durchführung der exemplifizierten Herstellung, Aufarbeitung und Zurückgewinnung dar:

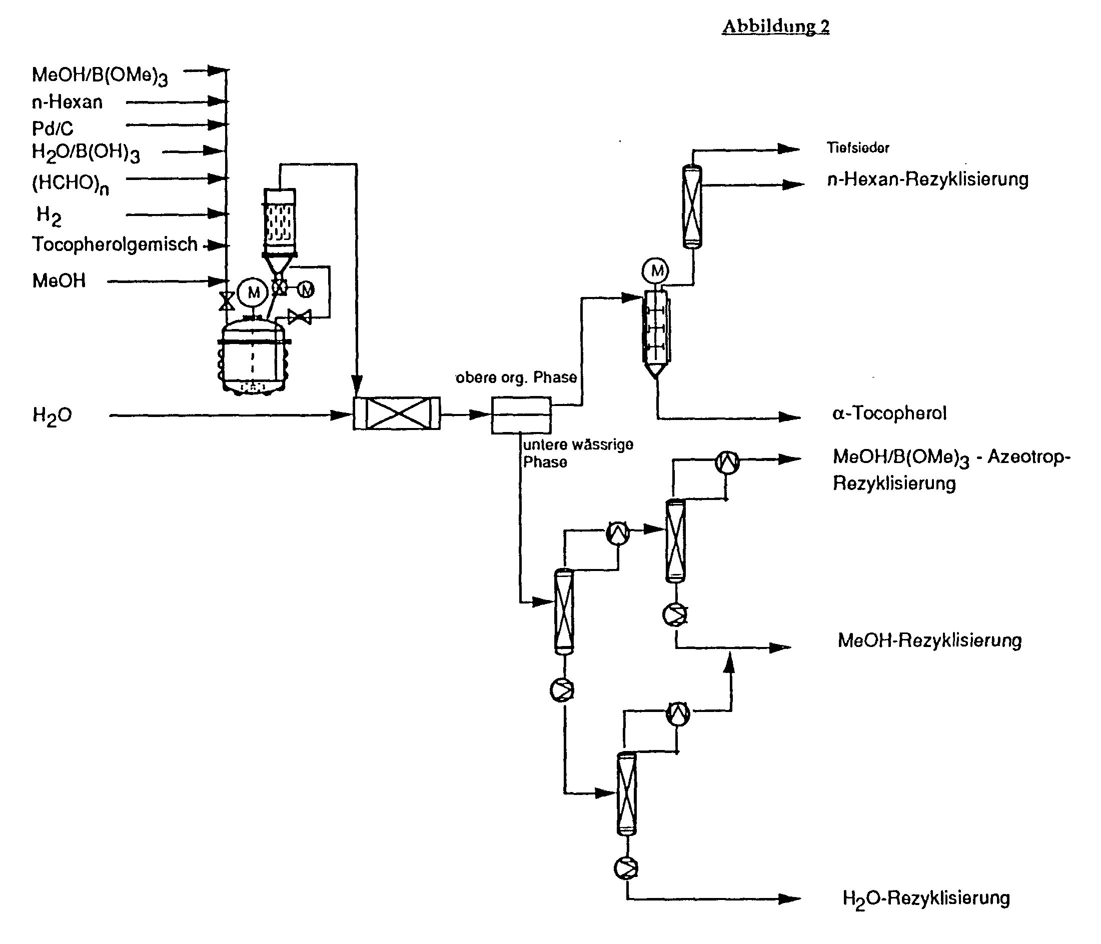

### Beispiel 4

### Reaktiv-Destillation zur Recyclisierung der Lösungsmittel und Bor-Reagenzien

Zur Untersuchung der Durchführbarkeit einer kontinuierlichen Destillation der Lösungsmittel mit überlagerter Reaktion der Borsäure zu Trimethylborat wird eine Bodenkolonne aus Glas mit 50 mm Durchmesser eingesetzt. Die Kolonne besitzt 32 praktische Böden, und der Zulauf befindet sich in der Mitte der Kolonne.

Für alle Untersuchungen werden Gemische mit einer repräsentativen Qualität synthetisch hergestellt. Diese Gemische bestehen im Mittel aus 56 ma-% Methanol, 9 ma-% Borsäure und 35 ma-% Wasser. Der Zulaufstrom in die Kolonne beträgt 1000 g/h und das Rückfluss:Entnahme-Verhältnis am Kolonnenkopf wird auf 35:1 eingestellt. Nachdem die Kolonne den stationären Betriebszustand erreicht hat, werden mehrere Bilanzen über einen Zeitraum von jeweils 8 Stunden bzw. 7 Stunden durchgeführt. Daraus ergibt sich ein Kopfstrom zwischen 240 und 242 g/Stunde und entsprechend ein Fusstrom zwischen 753 und 757 g/Stunde bei einem mittleren Bilanzfehler von 0,29%.

Die Analysen der Kopfprodukte erfolgen für die Methanolbestimmung mittels GC-interner Standard und für die Trimethylboratbestimmung mittels Borsäure-Titration mit Mannitol. Zudem wird eine Dichtekurve für den gesamten Konzentrationsbereich von Trimethylborat/Methanol aufgenommen. Dadurch ist es möglich, durch eine einfache Dichtebestimmung die Kopfkonzentration zu bestimmen. Beide Methoden liefern im Rahmen der Analysengenauigkeit identische Ergebnisse. Die Konzentration der Fussprodukte wird für Methanol ebenfalls mittels GC-interner Standard und für Wasser mittels Karl-Fischer-Titration bestimmt. Zusätzlich wird die nicht umgesetzte Borsäure, die als Fussprodukt die Anlage verlässt, mittels Mannitol-Titration ermittelt.

Die Analysenergebnisse zeigen auf, dass die Borsäure in der Kolonne zu ≥98,5% zu Trimethylborat umgesetzt werden kann. Dieses verlässt die Kolonne als Kopfprodukt mit einer Konzentration zwischen 59,5 und 62 ma-%, und der Rest besteht aus Methanol. Als Fussprodukt erhält man ein Gemisch, das aus ca. 57 ma-% Wasser und 43 ma-% Methanol besteht. Zusätzlich findet man noch etwa 0,18 ma-% nicht umgesetzte Borsäure.

Da das Kopfprodukt im Vergleich zu dem bei der Hydroxymethylierung eingesetzten Material zuviel Methanol enthält, muss es mittels weiterer Rektifikation auf in etwa azeotrope Zusammensetzung gebracht werden. Dies wird in einer zweiten Glasbodenkolonne, ebenfalls mit 50 mm Durchmesser, durchgeführt. Diese Kolonne besitzt 73 praktische Böden. Die Einspeisung erfolgt hier am 16. Boden (von oben gezählt). Diese destillative Aufarbeitung ist an sich konventionell und wird daher nicht mehr eingehend erläutert. Als Kopfprodukte erhält man ein Gemisch mit 72-74 ma-% Trimethylborat und entsprechend 28-26 ma-% Methanol. Als Fussprodukt kann weitgehend reines Methanol abgezogen werden.

Dieselbe Kolonne wird ebenfalls zur Auftrennung des als Fussprodukt aus der Reaktivdestillationskolonne stammenden Methanol/Wasser-Gemisches eingesetzt. Auch diese Auftrennung ist eine typische Standardaufgabenstellung in der chemischen Industrie und bedarf keiner näheren Erläuterung. Die Durchführung ergibt eine weitgehend vollständige Auftrennung zwischen Methanol (Kopfprodukt) und Wasser (Fussprodukt).

## Patentansprüche

1. Verfahren zur Umwandlung von Nicht-α-Tocopherolen in α-Tocopherol durch Umsetzung mindestens eines Nicht-α-Tocopherols mit Formaldehyd oder einem Derivat davon in Gegenwart von Borsäure oder einem Derivat davon unter katalytisch reduzierenden Bedingungen, **dadurch gekennzeichnet, dass** man als Lösungsmittel mit der zusätzlichen Funktion eines Katalysators ein azeotropes Gemisch von Trimethylborat und Methanol sowie als weiteres Lösungsmittel ein nichtpolares organisches Lösungsmittel verwendet, und dass man die Umsetzung bei Temperaturen zwischen 130 und 180°C durchführt.

2. Verfahren nach Anspruch 1, **dadurch gekennzeichnet, dass** das Gewichtsverhältnis des Trimethylborats zum Methanol im azeotropen Gemisch 50:50 bis 75:25 beträgt.

3. Verfahren nach Anspruch 2, **dadurch gekennzeichnet, dass** das Gewichtsverhältnis 70:30 beträgt.

4. Verfahren nach einem der Ansprüche 1 bis 3, **dadurch gekennzeichnet, dass** das Moläquivalentenverhältnis Trimethylborat zu eingesetzten Gesamt-Tocopherolen 0,1:1 bis 10:1 beträgt.

5. Verfahren nach Anspruch 4, **dadurch gekennzeichnet, dass** das Moläquivalentenverhältnis 1:1 bis 4:1 beträgt.

6. Verfahren nach einem der Ansprüche 1 bis 5, **dadurch gekennzeichnet, dass** das weitere, nichtpolare organische Lösungsmittel ein niederes Alkan, ein Gemisch von Alkanen oder ein aromatischer Kohlenwasserstoff ist.

7. Verfahren nach Anspruch 6, **dadurch gekennzeichnet, dass** man als nichtpolares organisches Lösungsmittel n-Hexan verwendet.

8. Verfahren nach Anspruch 6 oder 7, **dadurch gekennzeichnet, dass** man pro Moläquivalent Gesamt-Tocopherole bis zu 10 Liter nichtpolares, organisches Lösungsmittel verwendet.

9. Verfahren nach einem der Ansprüche 1 bis 8, **dadurch gekennzeichnet, dass** man die Umsetzung bei Temperaturen zwischen 140 und 170°C, durchführt.

10. Verfahren nach einem der Ansprüche 1 bis 9, **dadurch gekennzeichnet, dass** man die Umsetzung in Gegenwart von Borsäure oder in Gegenwart von aus dem im Reaktionsgemisch vorhandenen Trimethylborat und zugesetztem Wasser in situ erzeugter Borsäure durchführt und zur Aufarbeitung des Gemisches das nach beendeter Reaktion verbleibende Gemisch mit Methanol behandelt, wobei sich die Borsäure im Methanol löst, die so erhaltene, gewünschtenfalls durch Spülungen des nichtpolaren organischen Lösungsmittels ergänzte, verbleibende Lösung vom festen Katalysator abfiltriert, welcher so isoliert und wiederverwendet werden kann, das (gesamte) Filtrat mit Wasser behandelt, nach Phasentrennung das nichtpolare organische Lösungsmittel aus der organischen Phase destillativ zur Wiederverwendung abtrennt, um das Reaktionsprodukt α-Tocopherol freizusetzen, und die hauptsächlich aus Methanol, Wasser und Borsäure bestehende wässrige Phase einer Reaktiv-Destillation unterwirft, um das bei der Destillation infolge der Veresterung von Borsäure mit Methanol erzeugte Trimethylborat als azeotropes Gemisch von Trimethylborat und Methanol abzutrennen und zusätzlich Methanol und Wasser einer Wiederverwendung zuzuführen.

## Claims

1. A process for the conversion of non-α-tocopherols into α-tocopherol by the reaction of at least one non-α-tocopherol with formaldehyde or a derivative thereof in the presence of boric acid or a derivative thereof under catalytically reducing conditions, **characterized by** using an azeotropic mixture of trimethyl borate and methanol as the solvent with the additional function of a catalyst as well as a non-polar organic solvent as an additional solvent and by carrying out the reaction at temperatures between 130 and 180°C.

2. A process according to claim 1, **characterized in that** the weight ratio of trimethyl borate to methanol in the azeotropic mixture amounts to 50:50 to 75:25.

3. A process according to claim 2, **characterized in that** the weight ratio amounts to 70:30.

4. A process according to any one of claims 1 to 3, **characterized in that** the mol equivalent ratio of trimethyl borate to total tocopherols used amounts to 0.1:1 to 10:1.

5. A process according to claim 4, **characterized in that** the mol equivalent ratio amounts to 1:1 to 4:1.

6. A process according to any one of claims 1 to 5, **characterized in that** the additional non-polar organic solvent is a lower alkane, a mixture of alkanes or an aromatic hydrocarbon.

7. A process according to claim 6, **characterized in that** n-hexane is used as the non-polar organic solvent.

8. A process according to claim 6 or 7, **characterized in that** up to 10 litres of non-polar organic solvent are used per mol equivalent of total tocopherols.

9. A process according to any one of claims 1 to 8, **characterized in that** the reaction is carried out at temperatures between 140 and 170°C.

10. A process according to any one of claims 1 to 9, **characterized in that** the reaction is carried out in the presence of boric acid or in the presence of boric acid generated in situ from the trimethyl borate present in the reaction mixture and added water and, for the working up of the mixture, the mixture remaining after completion of the reaction is treated with methanol, whereby the boric acid dissolves in the methanol, the thus-obtained remaining solution, optionally supplemented by non-polar organic solvent rinsings, is separated from the solid catalyst by filtration, which catalyst can be so isolated and reused, the (entire) filtrate is treated with water, after phase separation the non-polar organic solvent is separated by distillation from the organic phase for reuse in order to liberate the reaction product α-tocopherol, and the aqueous phase consisting mainly of methanol, water and boric acid is subjected to a reactive distillation in order to separate the trimethyl borate, produced in the distillation as a result of the esterification of boric acid with methanol, as an azeotropic mixture of trimethyl borate and methanol, and in addition to reuse the methanol and water.

## Revendications

1. Procédé pour la conversion de non-α-tocophérols en α-tocophérol par réaction d'au moins un non-α-tocophérol avec du formaldéhyde ou un dérivé de celui-ci en présence d'acide borique ou d'un dérivé de celui-ci, dans des conditions catalytiquement réductrices, **caractérisé en ce qu'**on utilise comme solvant, ayant la fonction supplémentaire d'un catalyseur, un mélange azéotropique de méthanol et de borate de triméthyle ainsi que, comme autre solvant, un solvant organique non polaire, et **en ce qu'**on effectue la réaction à des températures comprises entre 130 et 180°C.

2. Procédé selon la revendication 1, **caractérisé en ce que** le rapport pondéral du borate de triméthyle au méthanol dans le mélange azéotropique va de 50:50 à 75:25.

3. Procédé selon la revendication 2, **caractérisé en ce que** le rapport pondéral est égal à 70:30.

4. Procédé selon l'une quelconque des revendications 1 à 3, **caractérisé en ce que** le rapport d'équivalents molaires du borate de triméthyle aux tocophérols totaux utilisés va de 0,1:1 à 10:1.

5. Procédé selon la revendication 4, **caractérisé en ce que** le rapport d'équivalents molaires va de 1:1 à 4:1.

6. Procédé selon l'une quelconque des revendications 1 à 5, **caractérisé en ce que** l'autre solvant organique non polaire est un alcane inférieur, un mélange d'alcanes ou un hydrocarbure aromatique.

7. Procédé selon la revendication 6, **caractérisé en ce qu'**on utilise comme solvant organique non polaire le n-hexane.

8. Procédé selon la revendication 6 ou 7, **caractérisé en ce qu'**on utilise jusqu'à 10 litres de solvant organique non polaire par équivalent molaire de tocophérols totaux.

9. Procédé selon l'une quelconque des revendications 1 à 8, **caractérisé en ce qu'**on effectue la réaction à des températures comprises entre 140 et 170°C.

10. Procédé selon l'une quelconque des revendications 1 à 9, **caractérisé en ce qu'**on effectue la réaction en présence d'acide borique ou en présence d'acide borique engendré in situ à partir du borate de triméthyle présent dans le mélange réactionnel et d'eau ajoutée, et, pour le traitement final du mélange, on traite par du méthanol le mélange restant à la fin de la réaction, ce par quoi l'acide borique se dissout dans le méthanol, la solution restante ainsi obtenue, si on le désire complétée par des liquides de lavage du solvant organique non polaire, est séparée par filtration du catalyseur solide, qui peut être ainsi isolé et réutilisé, on traite par de l'eau le filtrat (total), après séparation des phases on sépare le solvant organique non polaire par distillation de la phase organique, pour la réutilisation, afin de libérer le produit de réaction α-tocophérol, et on soumet à une distillation réactive la phase aqueuse principalement constituée de méthanol, eau et acide borique, afin de séparer sous forme de mélange azéotropique de méthanol et borate de triméthyle le borate de triméthyle produit lors de la distillation par suite de l'estérification de l'acide borique par le méthanol et d'envoyer en outre le méthanol et l'eau à une réutilisation.
